# EUROPEAN PATENT APPLICATION

(11) **EP 2 815 779 A1**
(43) Date of publication of application: **24.12.2014**
(21) Application number: 13173202.6
(22) Date of filing: 21.06.2013
(51) Int. Cl.: A61M 5/315, A61M 5/31

(54) **Prefilled mixing syringe and associated methods**

(71) Applicant: TRD-Dimitrov Limited, Varna 9000 (GB)
(72) Inventor: Dimitrov, Toni, 9000 Varna (BG); Racheva, Eleonora, 9000 Varna (BG)

(57) **Abstract**

Modifications in the design of the conventional syringe are disclosed, for constructing dual substance prefilled mixing syringe. Total storage volume is extended by utilizing plunger body inner compartment (2). Vacuum activated valve (3), moulded as part of the plunger is assuring one-way flow. The process of aspiration and mixing is simplified to one-step procedure, driven by the vacuum created upon plunger withdrawal. With the prefilled mixing syringe embodiment, exact mixing dosage of the substances is achieved, and the time for solution preparation is significantly reduced.

## Description

### Technical Field

The present invention relates to design of a ready to use syringe with mixing and flush capabilities, prefilled with any two liquid/liquid or liquid/dry substances.

### Background Art

In a prior studies, mixing syringe is suggested to be made on the basis of conventional syringe, using Pressure actuated valve for multi-chamber syringe applications US 20120265171 A1 (Gale H. Thorne and Jr. Gale H. Thorne) Publication date Oct 18 2012. With the valve and both substances placed in the syringe barrel, the mixing process becomes limited by the available volume. Therefore such design might not be appropriate for large volumes (20-30 ml).

In another study, Syringe with flow control valves and associated methods EP 2544732 A1 (Shawn P. Fojtik) Publication date Jan 16, 2013, the flow control is achieved with at least a pair of (i.e., at least two) valves. However, it has a very complicated structure, with relatively large number of components.

### Summary of Invention

Ready to use prefilled mixing syringe embodiment, as a means for eliminating the disadvantages of the above-cited previous studies, is disclosed.

Based on modifications in the design and the construction of the conventional syringe, the main object of the disclosed invention is to provide simple design and inexpensive construction of a syringe, with proper separation, storage, mixing and flush capabilities. Constructed as dual compartment syringe, it comprises of a syringe barrel compartment (1) and a plunger body inner compartment (2), used as an additional compartment. Both compartments communicate through standard industrial type of vacuum activated one-way valve (3), integrated into the front-end (8) of the plunger body (4), moulded as part of it.

The first compartment of the prefilled mixing syringe is confined within the inner space of the hollow plunger body (4). The liquid substance (19) inside, is sealed between a closing member (6)-self-displaceable rubber disk, placed at the back-end (7) of the plunger body (4), and the plunger valve (3). The second compartment is defined by the syringe barrel (5) itself, where the luer cap (17) and the plunger front-end (8), hermetically seal the compartment. Providing the valve (3) is in closed-state, the compartments are kept totally disparate.

The construction of the syringe is such that as the plunger assembly (9) is moved backwards, the pressure in the syringe barrel compartment (1) is reduced to substantially vacuous. Upon reaching predetermined value, vacuum activated one-way valve (3) turns in opened-state. The liquid substance (19) from the plunger body inner compartment (2), facilitated by the negative pressure gradient, flows with ease trough the opened valve (3) filling the syringe barrel compartment (1), and mixing with the stored dry/liquid substance (18). The vacuum generated in the syringe barrel compartment (1) is compensated by the liquid substance (19) flow, upon valve opening. At the same time as the liquid substance (19) is expelled out of the plunger body inner compartment (2), the closing member (6) shifts forward to balance the vacuum in the compartment. The plunger body outer compartment (20) is not hermetically sealed, and communicates with the outside through vents (15) perforated at the back-end (7) of the plunger. With the plunger assembly (9) in backmost position, and pressures the compartments balanced, the valve (3) returns in closed-state. By pushing the plunger assembly (9) forward, with the valve (3) still in closed-state, the residual gas (10) is released, and the syringe with the solution is ready to be dispensed.

Removable stopper (13) attaches main syringe wings (11) and plunger back-end (7) to prevent unintended plunger withdrawal/push up.

The prefilled mixing syringe embodiment can be of the size and volume of any standard conventional syringe, and any non standard size and volume syringe;
Similar in size, shape and volume with the conventional syringe for daily use, the disclosed mixing syringe embodiment is easy to operate, and can be used in convenient manner;
Dual compartment assembly of the prefilled mixing syringe embodiment reduces the time for preparation of any solution through simplifying the process of aspiration, mixing and flush. Prefilled mixing syringe embodiment guarantees that right substances (antibiotics, allergy medications, etc) with the exact dosage are to be used in the preparation of the solution;

### Brief Description of Drawings

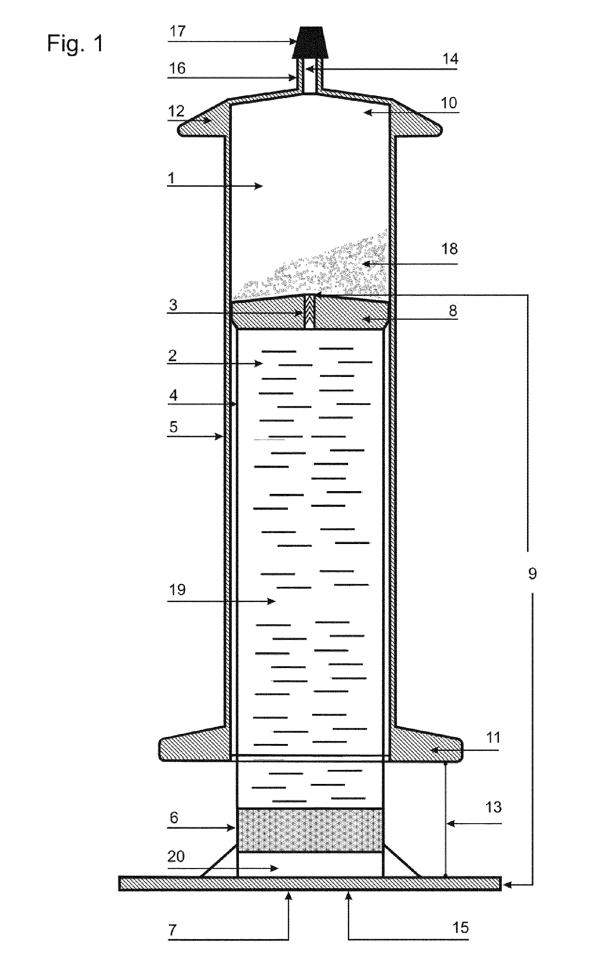

Fig. 1 Horizontal sectional view of a prefilled mixing syringe for preparation of solution of any two dry/liquid or liquid/liquid substances;

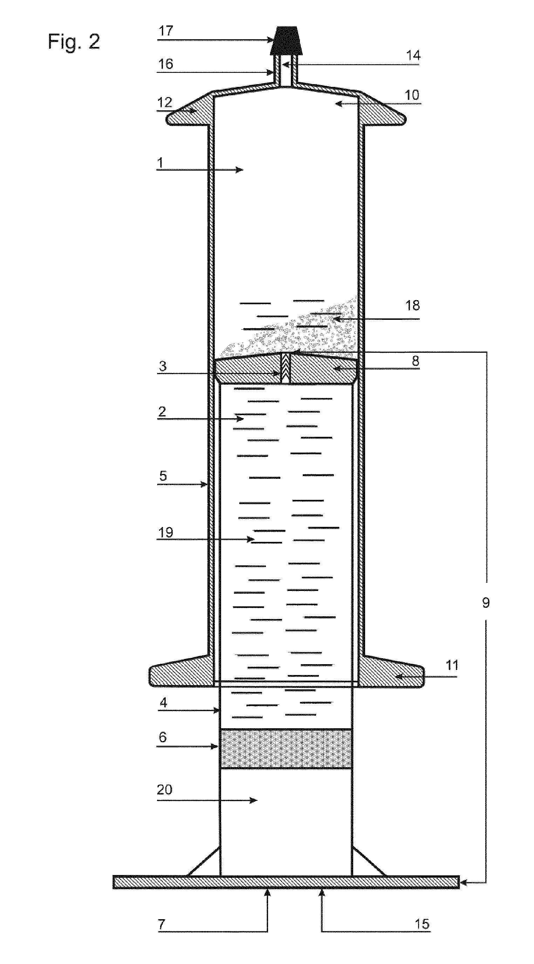

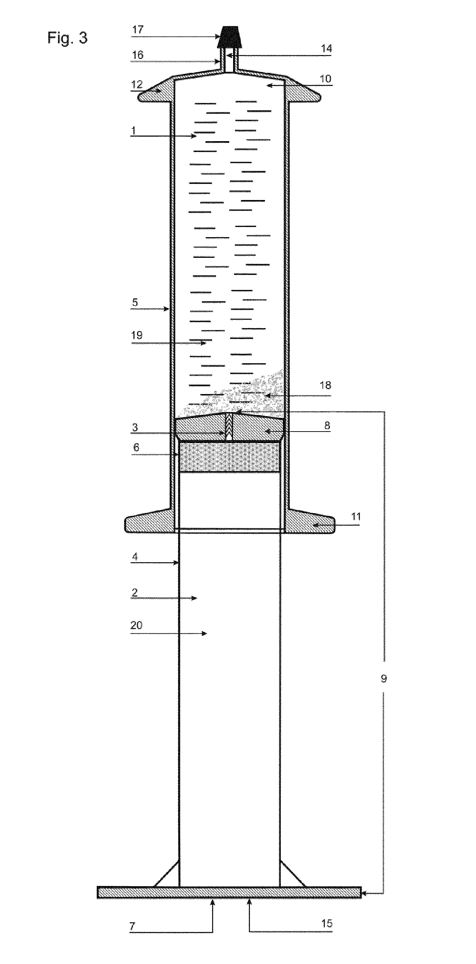

Fig. 2 and Fig. 3 Illustrative views showing how the liquid substance (19) is dispensed from the plunger body inner compartment (2) upon plunger assembly (9) withdrawal, as well as how the closing member (6), ensures complete wipe of the liquid substance (19) stored in the plunger body inner compartment (2);

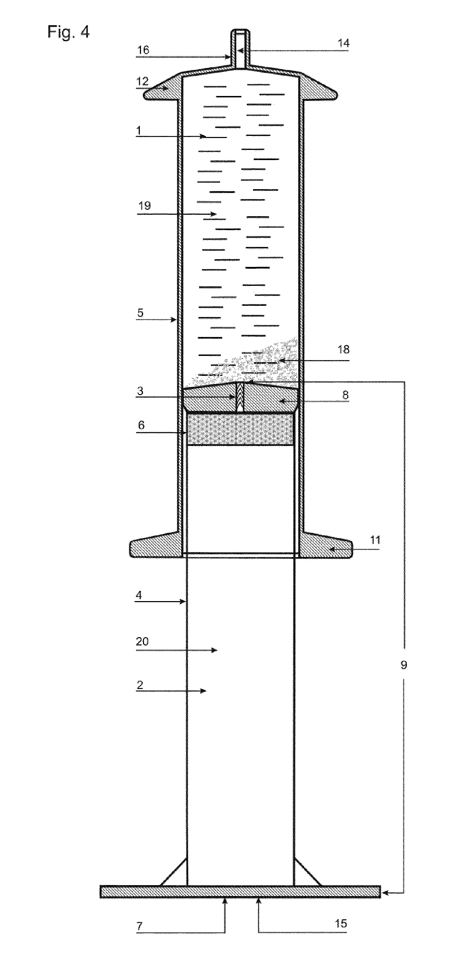

Fig. 4 View illustrating mixing syringe with solution ready for dispense-the luer cap (17) and the removable stopper (13) are detached;

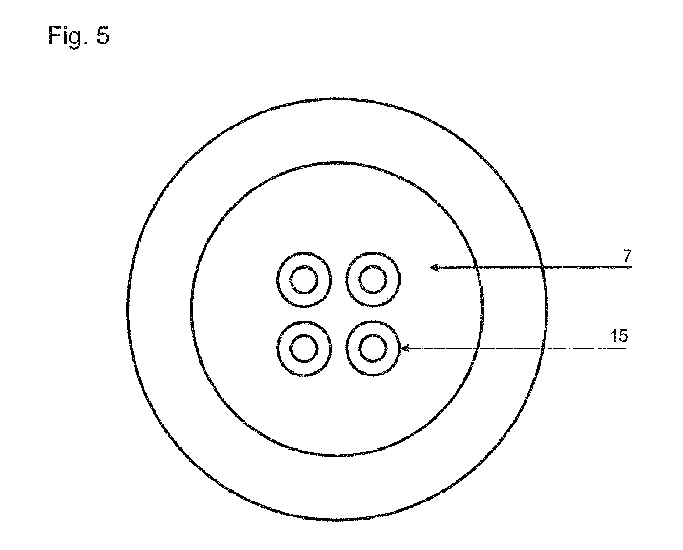

Fig. 5 Frontal view of the vents (15) perforated at the back-end (7) of the plunger body (4).

### Description of Embodiments

The main embodiment of the prefilled mixing syringe is to use the syringe marked with easily recognizable colour sign for given solution (e.g. Thiopental solution). This will eliminate unintended mistakes in emergency situation, ensuring the exact dosage of the right substances are prefilled in advance, (e.g. of any antibiotics, allergy medications, etc.);
In another embodiment, dual compartment assembly of the prefilled mixing syringe can be used to reduce significantly the time for solution preparation through simplifying the process of aspiration, mixing and flush;

### Industrial Applicability

Syringes with such design, are useful in a variety of methods and applications including, but not limited to mixing of any two dry/liquid or liquid/liquid substances, having different volumes and different mixing ratios;
The prefilled mixing syringe embodiment can be of the size and volume of any standard conventional syringe, including, but not limited to it, as well as it can be implemented in any non standard size and volume;
Similar in size, shape and volume with the conventional syringe for daily use, the disclosed mixing syringe embodiment is easy to operate, and can be used in convenient manner;

### Reference Signs List

1. Syringe barrel compartment
2. Plunger body inner compartment
3. Vacuum activated one-way valve
4. Plunger body
5. Syringe barrel
6. Closing member (self-displaceable rubber disk)
7. Plunger back-end
8. Plunger front-end
9. Plunger assembly
10. Gas
11. Main syringe wings
12. Additional tiny front wings
13. Removable stopper
14. Syringe orifice
15. Vents
16. Luer
17. Luer cap
18. Dry/liquid substance
19. Liquid substance
20. Plunger body outer compartment

### Citation List

Pressure actuated valve for multi-chamber syringe applications US 20120265171 A1 (Gale H. Thorne and Jr. Gale H. Thorne) Publication date Oct 18,2012.

Syringe with flow control valves and associated methods EP 2544732 A1 (Shawn P. Fojtik) Publication date Jan 16, 2013

## Claims

1. **Design and construction of Plunger assembly (9) that comprises:** Elongated plunger body (4), shaped as hollow tube, for storing liquid substance (19); plunger front-end (8), made of low friction soft plastic material, sized and shaped to wipe the substance from the syringe barrel compartment (1); standard industrial type of vacuum activated one-way valve (3), embedded into the front-end (8) of the plunger; closing member (6) - self-displaceable rubber disk, inserted into the plunger body (4), made of low friction light plastic material, impervious to liquids, sized and shaped to passively follow the liquids upon their disposal from the plunger body inner compartment (2); vents (15) perforated at the back-end (7) of the plunger, permitting the air to enter into the plunger body outer compartment (20); removable stopper (13) securing unintended plunger withdrawal/insertion;

2. **Extending the storage volume of the mixing syringe embodiment by utilizing the volume of the hollow plunger body (4), e.g. introducing the plunger body inner compartment (2);**

3. **Design and construction of an assembly of a prefilled mixing syringe that comprises:** The Plunger assembly (9) of Claim 1 and the storage volume of the plunger body (4) of Claim 2 and a Syringe barrel (5)-elongated hollow tube with circular inner surface and constant diameter, having syringe barrel compartment (1), for storing the dry/liquid substance (18); front-end with a luer (16), through which the fluid is dispensed, sealed by a luer cap (17); additional tiny front wings (12) appended to the front-end of the syringe barrel for stable grip;

4. **Dual compartment assembly of a prefilled mixing syringe as described in Claim 1, Claim 2 and Claim 3 that forms the communicating pathway of the mixing syringe embodiment, with diverse applications and variety of methods including, but not limited to mixing of any two dry/liquid or liquid/liquid substances, having different volumes and different mixing ratios;**

5. **Dual compartment assembly of a prefilled mixing syringe as described in Claim 1, Claim 2, Claim 3 and Claim 4, that can be of the size and volume of any standard conventional syringe, including, but not limited to it, as well as it can be implemented in any non standard size and volume;**

6. **A mixing method for the application of the prefilled mixing syringe of Claim 1, Claim 2, Claim 3, Claim 4 and Claim 5 comprising:** Embedding vacuum activated one-way valve (3) into the front-end (8) of the plunger, to ensure one-way flow of the liquid substance in the prefilled mixing syringe; Inserting closing member (6) - self-displaceable rubber disk, at the back-end (7) of the plunger body (4), to ensure complete wipe of stored liquid substance; Using the vacuum activated one-way valve (3) and the closing member (6) to secure the first compartment in the plunger body (4) of the prefilled mixing syringe; Affixing luer cap (17) to seal the syringe orifice (14); Attaching the plunger assembly (9), to secure the second compartment of the prefilled mixing syringe; The valve (3) closed-state keeps both compartments disparate, providing long-term proper separation and storage of the prefilled substances; Loading in advance, both liquid/liquid or liquid/dry substances necessary for solution preparation in the compartments; The liquid substance, stored in the plunger body inner compartment (2) fills entirely the inner space, so that the mixing syringe can be positioned either way during plunger assembly (9) withdrawal; The dry/liquid substance (18), stored in the syringe barrel compartment (1) does not fill entirely the inner space, assuring ease of mixture with the liquid substance (19); Detaching the removable stopper (13), to allow plunger assembly (9) withdrawal/insertion; Applying force to withdraw the plunger assembly (9) backwards, decrease the pressure in the syringe barrel compartment (1) to predetermined level, so that the vacuum activated one-way valve (3) turns in open-state; Dispensing the liquid substance (19) from the plunger body inner compartment (2), simultaneously decrease the pressure in the compartment. The closing member (6), passively shifting towards the valve (3), ensuring complete wipe of the liquid substance stored in the plunger body inner compartment (2); Along with shifting of the closing member (6), the air flow enters into the plunger body outer compartment (20) through the vents (15), perforated at the back-end (7) of the plunger body (4), to compensate the resulting vacuum in the plunger body outer compartment (20); Removing the luer cap (17) to purge any gas (10) from the syringe barrel compartment (1); in this manner the gas is eliminated and the syringe with the solution is ready for dispense; Dispensing the resultant solution;

7. **Prefilled mixing syringe for preparation of Thiopental solution, comprising prefilled mixing syringe of Claim 1, Claim 2, Claim 3, Claim 4 and Claim 5 and a mixing method of Claim 6:** Thiopental Sodium is general anaesthetic agent, usually given by intravenous injection. The active ingredient is Thiopental Sodium 500 mg, powder for solution. Because of low chemical stability of Sodium Thiopental after preparation, manufacturer's instructions recommend discarding unused portions 24 h after reconstitution, which daily results in large volumes of prepared thiopental disposal. To overcome the abovementioned disadvantages and provide the public with useful commercial choice, it is further object of the invention to provide a prefilled mixing syringe for Thiopental solution preparation, as described below:
a) 500 mg Thiopental powder is placed in the syringe barrel compartment (1); 20 ml. Normal Saline 0.9 % is placed in the plunger body inner compartment (2);
b) Dispensing the Normal Saline from the plunger body inner compartment (2) into the syringe barrel compartment (1) containing Thiopental powder, upon plunger assembly (9) withdrawal;
Mixing of the resultant solution and purging the gas from the syringe barrel compartment (1).

8. **Prefilled mixing syringe of any size and volume, marked with colour sign, for preparation of solution of any two dry/liquid or liquid/liquid substances, comprising prefilled mixing syringe of Claim 1, Claim 2, Claim 3, Claim 4 and Claim 5 and a mixing method of Claim 6.**
